# EUROPEAN PATENT SPECIFICATION

(11) **EP 2 459 145 B1**
(45) Date of publication and mention of the grant of the patent: **01.03.2017**
(21) Application number: 10770957.8
(22) Date of filing: 27.07.2010
(51) Int. Cl.: A61H 33/02

(54) **BABY BATH, NAMELY FOR TODDLERS AND INFANTS**
BABYBAD, NÄMLICH FÜR SÄUGLINGE UND KLEINKINDER
BAIGNOIRE POUR BÉBÉS ET ENFANTS EN BAS ÂGE

(30) Priority: 27.07.2009 CZ 20090493
(43) Date of publication of application: 06.06.2012
(73) Proprietor: Koller, Rudolf, 1100 Wien (AT)
(72) Inventor: Koller, Rudolf, 1100 Wien (AT)
(74) Representative: Kratochvil, Vaclav
(86) International application number: PCT/CZ2010/000083
(87) International publication number: WO 2011/012094

(56) References cited:
- EP-A1- 1 245 216
- EP-A2- 0 252 435
- DE-U1- 20 000 375
- US-A- 5 604 940

## Description

### Technical field

The patent relates to the baby bath, namely for toddlers and infants between 500 mm and 1200 mm of height...

### Background of the Invention

There are baby baths for toddlers and infants. Usually these baths are about 100 cm long and made of various plastic materials. Their disadvantage is they have to be separately filled with water which needs to be kept at certain temperature. During bathing the water remains still and does not have any effect on baby's body.

We also know hydro-massage and air-massage baths. These are bath tubs of standard and above standard sizes equipped with hydro-massage and/or air-massage system terminated with jets outfalling in various parts of the bath tub. Their disadvantage is that these tubs are too big, unsuitable for infants and children not only due to size reasons but also due to high pressure of outlet water speed and air from jets and due to safety of bathed infant or toddler.

### Disclosure of the invention

Above stated disadvantages are on certain level solved by the baby bath, namely for infants and toddlers between 500mm and 1200 mm of height. Its principle as claimed is that this bath is provided with at least four jets connected with a hydro-massage system and at least five jets connected with an air-massage system, the jets have outlet openings of the size 0,5 to 20mm, connected to a water pump with the output of 20 to 150 W and/or to an ventilator with the output of 4 to 60 W. In case of bigger jets, the outlet opening may be assembled from several jets with smaller diameter.

The baby bath may be equipped with halogen lights and/or LEDs and possibly with at least one inlet of aromatherapeutic agent.

Bottom of the baby bath can be inside provided with anti-slip surface and outlet. The baby bath with advantage is provided with water heating. The fan has a vibration advantage.

With regards to its size between 500 and 1200 mm the baby bath is suitable in particular for toddlers and infants and can be easily transported to any place. As the bath is provided mainly with jets with relatively small outlet opening or possible bigger opening with several smaller diameter jets connected to the water pump and/or air ventilator, the outlet media is suitable for infants and small children having a calming effect and providing massage at the same time thus securing blood perfusion in parts of the body that can reached with difficulties during bathing.

As the bath is provided with at least four jets connected to the hydro-massage system and/or with at least five jets connected to the air-massage system it is possible that the effluent water can be directed to any part of the body with minimum movements and the massage effect can be selected according to child's requirements.

The baby bath can be equipped with lighting, in particular halogen lights and/or LEDs. This lighting allows use of the baby bath also in the area with lower intensity of surrounding light and may sweeten the bathing moment with help of various light effects. Aromatherapeutic agent inlet simplifies dosing of bath additives.

The baby bath can have other additional adjustments such as inside bottom provided with anti-slip surface preventing slipping of the baby under the water level. Also the baby bath can be provided with baby couch, bolster or have other shaping allowing better position of the baby.

In case the baby bath bottom is provided with the outlet, it is not necessary to carry full bath or remove water step by step but the water can be let out to relevant containers.

The baby bath can be equipped with heating allowing keeping of required temperature during the whole bathing time.

Advantage of this solution is that volume of air and/or water ejected by aggregates is several times lower compared to classic bath tubes, therefore the medium does not whirl water so much and does not reduce its temperature.

Used vibration ventilator is in effect noiseless compared to relatively noisy rotation ventilators used for common bath tubs and also the pump is silent in the given version.

Substantial advantage of this solution is mobility of the bath tub with the system contrary to hydro-massage and air-massage standard size tubs, which are only stationary. As the device is not stationary and permanently fixed, the baby bath is easy to be maintained as it is easy to empty the remaining water from the hydro-massage system.

The baby bath of this solution represents simple system with low input and easy maintenance with minimum power demands and almost zero negative environmental impact.

Hydro-massage and air-massage system brings positive therapeutic effect and contributes to maximum hygiene`during bathing of infants. Technical solution of electrical installation is in accordance with safety standard prescribed by relevant European standards. Baby baths also meet electromagnetic compatibility requirements. Maintenance demands are simple and do not require any extraordinary steps. Air-massage system can be only purged after the bath emptying with air from the ventilator. Thus the air system gets rid of the water and possible imparities. Massage system can be flushed with water flow from manual shower and turning the baby bath upside down will empty the remaining water from the system. With respect to the purpose of the bath for toddlers and infants without assumption of use of irritating cosmetic agents and with respect to described maintenance, the use of baby baths with hydro-massage and air-massage system represent almost no environmental load. In case of the use of ergonomically shaped baths it is possible to install jets in parts contributing to positive effect of a massage bath. Advantage of baths with outlet opening is the easier manipulation.

### Overview of pictures on drawings

Solution according to this patent will be in more detail described on specific examples with help of attached drawings. Picture 1 shows the example elevation of baby bath. The picture 2 shows it in profile elevation. Picture 3 shows it in section.

### Examples of patent execution

Baby bath **1** for toddlers and infants is 950 cm long, 550 mm wide and 240 mm deep. It is equipped on the circumference with four jets **2** connected with hydro-massage system and five jets **3** connected to air-massage system, where jets **2**, **3** have the outlet opening size of 0,6 mm and are connected to the water pump **4** with the output 90 W and to the air ventilator **5** with the output 6 W. Peripheral wall is also provided with lighting formed by light chain with ten LED lights. **6**. The light chain is illuminating continuously the bathing area of the baby bath **1**.

The baby bath **1** can be provided with non-demonstrated inlet of aromatherapeutic agent connected with the container. Bath bottom **1** is inside provided with anti-slip surface and outlet.

The baby bath **1** may be equipped with heating, the source of which **7** is located near other devices as are pump **4**, ventilator **5**, electrical wires etc.

In other version the baby bath **1** can be provided with lighting created by pair of halogen lights.

Air-massage jets **3** are located equally on the bath bottom **1** aiming to reach even massage effect. Hydro-massage jets **2** are placed in such a way, that four jets **2** point at side parts, remaining jets **2** point at soles.

Massage systems and lighting are switched on by remote electronic control allowing apart from switching on and off the massage or light chain other additional functions as cycling, control of massage effect etc.

### Industrial usability

Baby bath according to this patent can be used namely for bathing of toddlers and infants in households but also in hospitals, medical institution etc.

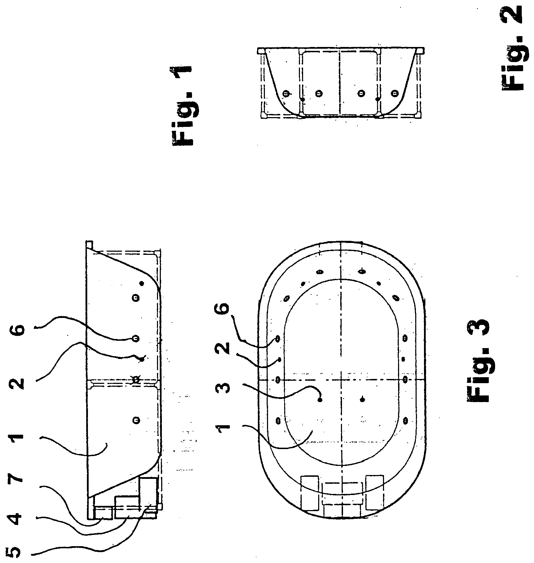

## Claims

1. The baby bath for toddlers and infants between 500 and 1200 mm of size is **characterized by** at least four jets (2) connected with a hydro-massage system and at least five jets (3) connected with an air-massage system, the jets (2, 3) have outlet openings of the size 0,5 to 20 mm, connected to a water pump (4) with the output 20 to 150 W and/or to an air ventilator (5) with the output 4 to 60 W.

2. The baby bath according to claim 1 is **characterized by** lighting created by halogen light and/or LED lights (6).

3. The baby bath according to any above claims is **characterized by** at least one inlet of aromatherapeutic agent.

4. The baby bath according to any above claims is **characterized by** the inside bottom provided with anti-slip surface.

5. The baby bath according to any above claims is **characterized by** the outlet in the bottom.

6. The baby bath according to any above claims is **characterized by** heating.

7. The baby bath according to any above claims is **characterized by** a vibration ventilator (5).

## Patentansprüche

1. Das Wännchen, insbesondere für Kleinstkinder und Säuglinge, dessen Länge von 500 bis 1200 mm beträgt, **dadurch gekennzeichnet, dass** es mindestens vier Düsen (2), verbunden mit dem Hydromassage-System, und mindestens fünf Düsen (3), verbunden mit dem Luftmassage-System, einschließt, wobei die Düsen (2, 3) die Austrittsöffnungen mit der Grösse von 0,5 bis 20 mm haben und an der Wasserpumpe (4) mit der Leistung von 20 bis 150 W und/oder am Lüfter (5) mit der Leistung von 4 bis 60 W angeschlossen werden.

2. Die Babybadewanne nach dem Anspruch 1, **dadurch gekennzeichnet, dass** sie mit der Beleuchtung ausgerüstet ist, die mit der Halogenlampe und/oder LEDs (6) gebildet wird.

3. Die Babybadewanne nach jedwedem der angeführten Ansprüche, **dadurch gekennzeichnet, dass** sie mit mindestens einer Zuführung des Aromatherapie-Stoffs ausgerüstet ist.

4. Die Babybadewanne nach jedwedem der angeführten Ansprüche, **dadurch gekennzeichnet, dass** deren Boden auf der Innenseite mit rutschfester Oberfläche ausgerüstet ist.

5. Babybadewanne nach jedwedem der angeführten Ansprüche, **dadurch gekennzeichnet, dass** der Boden mit dem Auslass ausgerüstet ist.

6. Die Babybadewanne nach jedwedem der angeführten Ansprüche, **dadurch gekennzeichnet, dass** sie mit der Erwärmung ausgerüstet ist.

7. Die Babybadewanne nach jedwedem der angeführten Ansprüche, **dadurch gekennzeichnet, dass** der Lüfter (5) vibriert.

## Revendications

1. La baignoire, surtout pour bébés et nourrissons, longue de 500 à 1200 mm, **se caractérisant par** quatre lances d'eau (2) au moins, raccordées à un système hydromasseur, et par cinq lances d'eau (3) au moins, raccordées à un système aéromasseur, les lances d'eau (2, 3) ayant les orifices de sortie de 0,5 à 20 mm et étant raccordées à une pompe d'eau (4) à la puissance de 20 à 150 W, et/ou à un aérateur (5) à la puissance de 4 à 60 W.

2. La baignoire selon la revendication 1, **se caractérisant par** un éclairage lampe halogène et/ou diodes LED (6).

3. La baignoire selon une des revendications mentionnées, **se caractérisant par** au moins une alimentation en substance aromathérapique.

4. La baignoire selon une des revendications mentionnées, **se caractérisant par** un fond pourvu de surface anti-glisse sur sa face intérieure.

5. La baignoire selon une des revendications mentionnées, **se caractérisant par** un fond pourvu d'orifice de vidange.

6. La baignoire selon une des revendications mentionnées, **se caractérisant par** un chauffage.

7. La baignoire selon une des revendications mentionnées, **se caractérisant par le fait que** son aérateur (5) est vibrant.
